# EUROPÄISCHE PATENTANMELDUNG

(11) **EP 1 205 544 A2**
(43) Veröffentlichungstag der Anmeldung: **15.05.2002**
(21) Anmeldenummer: 01124390.4
(22) Anmeldetag: 25.10.2001
(51) Int. Cl.: C12N 9/20, C07D 307/12, C07D 309/10

(54) **Verfahren zur Herstellung von Linalooxid oder von Linalooloxid enthaltenden Gemischen**

(30) Priorität: 07.11.2000 DE 10055092
(71) Anmelder: HAARMANN & REIMER GMBH, 37601 Holzminden (DE)
(72) Erfinder: Hilmer, Jens-Michael, Dr., 37671 Höxter (DE); Gatfield, Ian-Lucas, Dr., 37671 Höxter (DE)
(74) Vertreter: Mann, Volker, Dr.

(57) **Zusammenfassung**

Die vorliegende Erfindung betrifft ein Verfahren zur Herstellung von Linalooloxid oder von Linalooloxid enthaltenen Gemischen, bei dem Linalool oder Linalool enthaltene Gemische in Gegenwart von Enzymen oxidiert werden.

## Beschreibung

Die vorliegende Erfindung betrifft ein Verfahren zur Herstellung von Linalooloxid oder von Linalooloxid erhaltenden Gemischen.

Bei Linalooloxid handelt es sich um einen lange bekannten Aromastoff, der in einer Vielzahl von Aromen Einsatz findet. Linalooloxid kommt dabei zum einen als Fünfring in der furanoiden Form vor (2-Methyl-2-vinyl-5-(α-hydroxyisopropyl)tetrahydrofuran, CAS 60047-17-8; cis und trans: CAS 5989-33-3 und 34995-77-2), zum anderen als 6-Ring in der pyranoiden Form (2,2,6-Trimethyl-6-vinyltetrahydro-2H-pyran-3-ol, CAS 14049-11-7; cis und trans: CAS 14009-71-3 und 39028-58-5). Er kommt von Natur aus in etherischen Ölen wie Rosenholzöl und Osmanthus abs. und Früchten vor (Volatile Compounds in Food, Qualitative and Quantitative Data, 7. Auflage, 1996, TNO Nutrition and Food Research Institute, Zeist, Niederlande) und weist einen blumig-erdigen, leicht bergamotartigen Geruch auf (Bauer, Garbe, Surburg, Common Fragrance and Flavor Materials, zweite Auflage, VCH, Weinheim 1990, S. 114). Der Geschmackseindruck von Linalooloxid geht in die Richtung Citrus, Aprikose, Heidelbeere, blumig.

Der Fachmann unterscheidet im Bereich der Aromen drei Bereiche: natürliche, naturidentische und künstliche Aromen. Künstliche Aromen sind nur von geringerem Interesse, da die Akzeptanz beim Verbraucher nicht besonders hoch ist. Ein Beispiel für die Herstellung eines naturidentischen Aromastoffes ist in der JP A 59225176 beschrieben. Gegenstand dieser Veröffentlichung ist die Produktion von Linalooloxid. Hierbei werden durch chemische Synthese nicht natürliche Ausgangsstoffe unter Verwendung von starken Mineralsäuren zu Linalooloxid umgesetzt. Ein solches Produkt kann nur als "naturidentisch", jedoch nicht als natürlich eingestuft werden und darf daher nicht zur Herstellung von natürlichen Aromen verwendet werden.

Am hochwertigsten sind die natürlichen Aromen und damit auch die darin enthaltenen natürlichen Aromastoffe einzustufen. Dies zeigt sich auch deutlich an den wesentlich höheren Preisen, die sich mit natürlichen Aromen erzielen lassen. Zudem steigt das Interesse der Verbraucher an natürlichen Produkten und damit auch an natürlichen Aromen, die für deren Herstellung benötigt werden.

Daher ist die Aromaindustrie bestrebt, natürliche Bausteine für natürliche Aromen in ausreichender Menge und Qualität zur Verfügung zu haben. Allerdings ist die Isolierung dieser einzelnen Bausteine aus Naturextrakten in der Regel nicht wirtschaftlich zu realisieren.

Ein Beispiel für die fermentative Herstellung von Linalooloxid ist aus Demyttenaere (Demyttenaere, J.C.R., 1998, Biotransformation of terpenes by füngi for the production of bioflavors, Meded. - Fac. Landbouwkd. Toegepaste Biol. Wet., 63 (4a), 1321-1324, und Demyttenaere, J.C.R.; Willemen, H.M., 1998, Biotransformation of linalool to furanoid and pyranoid linalool oxides by Aspergillus niger, Phytochemistry, 47 (6),1029-1036) bekannt. Dort wird die Biotransformation von Terpenalkoholen mit Hilfe von Aspergillus niger- und Penicillium-Stämmen beschrieben. Dieses Verfahren ist jedoch sehr aufwendig in der Durchführung, sehr langsam in der Umsetzung und führt lediglich zu Ausbeuten in Höhe von 20 - 33 % an Linalooloxid.

Weiterhin ist der Einsatz von Enzymen zur Epoxydierung von Doppelbindungen in Fettsäuren und Alkenen bekannt (**1**. PCT WO 91/04333. **2.** Jarvie, A.W.P.; Overton, N.; St. Pourcain, C.B., 1998, Enzymatic epoxidation of polybutadiene, Chem. Comm., 177-178. **3**. Lemoult, S.C.; Richardson, P.F.; Roberts, S.M., 1995, Lipase catalysed Baeyer-Villiger Reactions, J. Chem. Soc. Perkin Trans. I, 89-91. **4**. Rüsch, M.; Warwel, S., 1996, Peroxy fatty acids: lipase catalysed preparation and epoxidation, Lipid Technology, 7, 77-80. **5**. Miurra, Y.; Yamane, T., 1997, Epoxidation of alkanes and cycloalkanes by microbial lipases immobilized with photo crosslinkable resin prepolymer, Biotechnol. Lett., 19, 7, 611-613). Dabei kommt es zur Epoxidierung von Doppelbindungen analog der chemischen Reaktion über eine Peroxycarbonsäure. Hierbei katalysiert das Enzym Lipase (E.C. 3.1.1.3, CAS-Nr. 9001-62-1, z.B. von Pseudomonas cepacia, DSM 3959, Candida antarctica DSM 3855, DSM 3908, DSM 3909, Humicola lanuginosa, DSM 3819, DSM 4109, Humicola brevispora, DSM 4110, Humicola brevis var. thermoidea, DSM 4111, Humicola isolens, DSM 1800) in Gegenwart von Wasserstoffperoxid die intermediäre Bildung der Peroxycarbonsäure (WO 91/04333).

Aus zahlreichen Veröffentlichungen ist der Einsatz von Lipasen im Labor - oder Produktionsmaßstab bekannt, im Wesentlichen zu Veresterungen oder Umesterungen. In keiner dieser Beschreibungen wird jedoch die Herstellung von furanoidem oder pyranoidem Linalooloxid auf enzymatischem Wege beschrieben.

Aufgabe der vorliegenden Erfindungen ist es nunmehr, Linalooloxid oder ein Linalooloxid enthaltendes Gemisch zu erzeugen, das als natürlich eingestuft und zur Herstellung von natürlichen Aromen verwendet werden kann. Außerdem soll erreicht werden, dass die Ausbeuten deutlich höher als im bisherigen Stand der Technik liegen. Insbesondere soll das Ziel erreicht werden, Ausbeuten oberhalb von 90 % zu erzielen.

Es wurde ein Verfahren zur Herstellung von Linalooloxid oder von Linalooloxid enthaltenen Gemischen gefunden, das dadurch gekennzeichnet ist, dass Linalool oder Linalool enthaltende Gemische in Gegenwart eines Enzyms oxidiert werden.

In dem erfindungsgemäßen Verfahren können sowohl D-Linalool als auch L-Linalool zum Einsatz kommen. Diese Substanzen können in reiner Form oder in Form eines Gemisches vorliegen. Ebenso können die Linaloole im Gemisch mit weiteren Stoffen eingesetzt werden.

Als solche weiteren Stoffe kommen etherische Öle, Absolues oder Auszüge in Betracht. In einer bevorzugten Ausführungsform kann Linalool in etherischen Ölen vorliegen, wie Rosenholzöl, Corianderöl, Basilikumöl, Lavendelöl usw. Auch hier ist es möglich, dass D-Linalool oder L-Linalool als Einzelsubstanzen in dem Reaktionsmedium vorhanden sind. Ebenso kommt natürlich ein Gemisch aus D-Linalool und L-Linalool in dem Reaktionsmedium für die erfindungsgemäßen Zwecke in Betracht.

Bei den erfindungsgemäß eingesetzten Enzymen handelt es sich vorzugsweise um Hydrolasen, speziell Lipasen oder Proteasen. Besonders bevorzugt kommen erfindungsgemäß Lipasen in Betracht. Im besonderen wird immobilisierte Lipase bevorzugt, z.B. Chirazym C2 L2 der Firma Roche Diagnostics, Penzberg, Deutschland, oder Novozym 435 der Firma Novo Nordisk, Dänemark.

Vorzugsweise handelt es sich um Enzyme, die mit Hilfe folgender Mikroorganismen gewonnen werden können: Pseudomonas cepacia, z.B. DSM 3959, Candida antarctica z.B. DSM 3855, DSM 3908, DSM 3909, Humicola lanuginosa, z.B. DSM 3819, DSM 4109, Humicola brevispora, z.B. DSM 4110, Humicola brevis var. thermoidea, z.B. DSM 4111, Humicola isolens, z.B. DSM 1800.

Als Enzymproduzenten sind insbesondere Hefen bevorzugt. Hierfür kommen besonders bevorzugt Candida-Arten in Form von Reinkulturen oder Mischkulturen dieser Hefen in Betracht. Ganz besonders bevorzugt ist Candida antarctica. Für das erfindungsgemäße Verfahren höchst bevorzugt sind demgemäß Lipasen aus Candida antarctica.

Nach dem erfindungsgemäßen Verfahren wird die Oxidation von Linalool vorzugsweise in Gegenwart der genannten Enzyme und eines Oxidationsmittels durchgeführt. Besonders bevorzugt ist Wasserstoffperoxid. Die Oxidationsmittel können vor oder während des Oxidationsverfahrens zugesetzt werden. Ebenso ist es erfindungsgemäß möglich, das Medium derart zusammenzusetzen und/oder die Reaktionsbedingungen so zu wählen, dass sich während des Ablaufs des erfindungsgemäßen Verfahrens das gewünschte Oxidationsmittel bildet. Insbesondere kann die Oxidationsmittelbildung unter Einsatz von Enzymen durchgeführt werden. Beispiele hierfür sind Oxidasen, z.B. Glucoseoxidase. Ebenso ist auch der Einsatz nicht enzymatischer Katalysatoren für die Herstellung des Wasserstoffperoxids denkbar.

In einer erfindungsgemäßen Variante ist es somit z.B. möglich, die Zusammensetzung des Mediums und der Enzyme so zu wählen, dass während des Verfahrens in situ das Wasserstoffperoxid erzeugt wird. Dabei kann die Wasserstoffperoxiderzeugung vor Beginn des Oxidationsverfahrens gestartet werden oder man lässt die Wasserstoffperoxiderzeugung in situ während des Oxidationsprozesses ablaufen. Erfindungsgemäß kann beispielsweise das Wasserstoffperoxid enzymatisch gebildet werden, indem Glukose in wässrigem Medium mit dem Enzym Glucoseoxidase oxidiert wird.

Zusätzlich zu dem Einsatz von Enzymen und Oxidationsmittel kann der Reaktionslösung oft auch ein Lösungsmittel zugesetzt werden. Als Lösungsmittel kommen erfindungsgemäß Essigsäureester in Betracht, bevorzugt Essigsäureethylester. Außer den genannten Estern kommen auch Carbonsäuren in Betracht, die Peroxycarbonsäuren zu bilden vermögen. Beispiele hierfür sind Essigsäure, Ameisensäure, Benzoesäure. Besonders bevorzugt ist Essigsäure.

Vorzugsweise handelt es sich erfindungsgemäß um solche Lösungsmittel, die mit Hilfe der Enzyme in Anwesenheit von Wasserstoffperoxid Persäuren bilden können. Ein Beispiel hierfür ist Essigsäureethylester, der mit Hilfe von Lipasen in Anwesenheit von Wasserstoffperoxyd intermediär Peressigsäure bildet.

In einer erfindungsgemäß besonders bevorzugten Form läuft demgemäß folgende Reaktion ab:

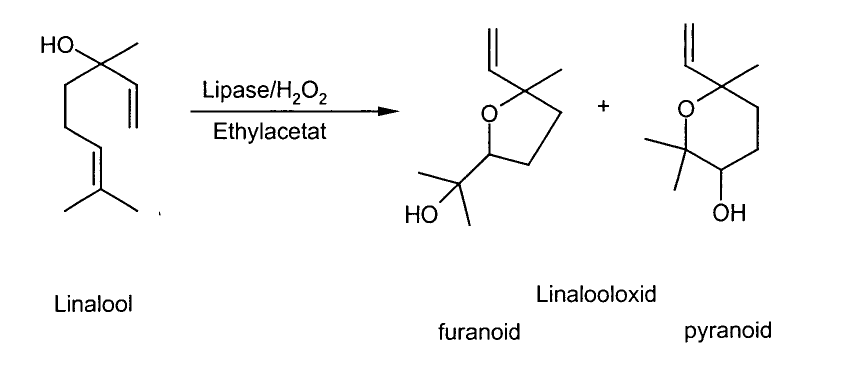

Mit Hilfe des beschriebenen erfindungsgemäßen Verfahrens ist es überraschend möglich, Linalooloxid herzustellen, das als natürlich eingestuft werden kann und daher zur Herstellung von natürlichen Aromen verwendet werden kann. Außerdem hat sich überraschender Weise ergeben, dass sich auf einfache Art und Weise natürliches Linalooloxid in Ausbeuten von mehr als 90 % erzeugen lässt. Damit weist das erfindungsgemäße Verfahren einen entscheidenden Vorteil gegenüber den bisher bekannten Fermentationsverfahren auf, die mit Hilfe von Mikroorganismen arbeiten, und eignet sich demgemäß zur industriellen Herstellung von natürlichem Linalooloxid.

Erfindungsgemäß kann das Linalooloxid direkt oder in Aromen für die verschiedensten Lebensmitteln zum Einsatz kommen. Dies ist insbesondere dort möglich, wo die Geschmacksrichtungen "Citrus", "Aprikose" und "Heidelbeere" erzeugt werden sollen.

### Beispiel

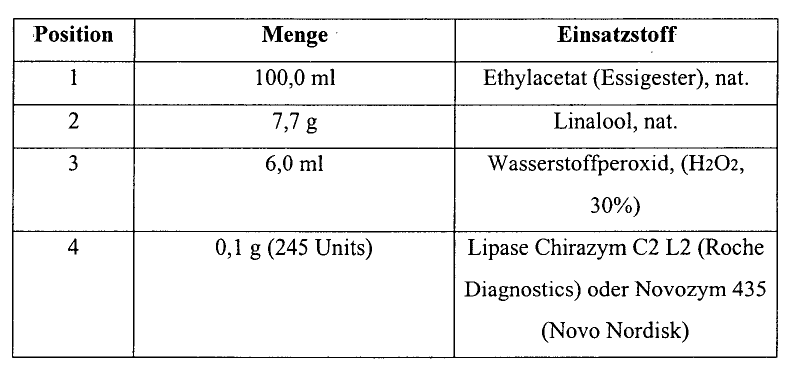

### Durchführung:

Ethylacetat (Pos. 1) wird in der 2 L-Dreihalskolbenrührapparatur mit Flügelrührer vorgelegt, Linalool (Pos. 2), Wasserstoffperoxyd (H₂O₂, 30 %ig, Pos. 3) und Enzym (Pos. 4) werden unter langsamen Rühren hinzugefügt. Es wird bei Raumtemperatur langsam gerührt (ca. 100 - 120 UpM). Prozessbegleitend wird alle 1-2 Tage per GC die Zusammensetzung analysiert.

### Ergebnis:

Bei den in der Tabelle aufgeführten GC-Ergebnissen wurden die Flächenprozente der Essigsäure herausgerechnet (außer bei der Essigsäure).

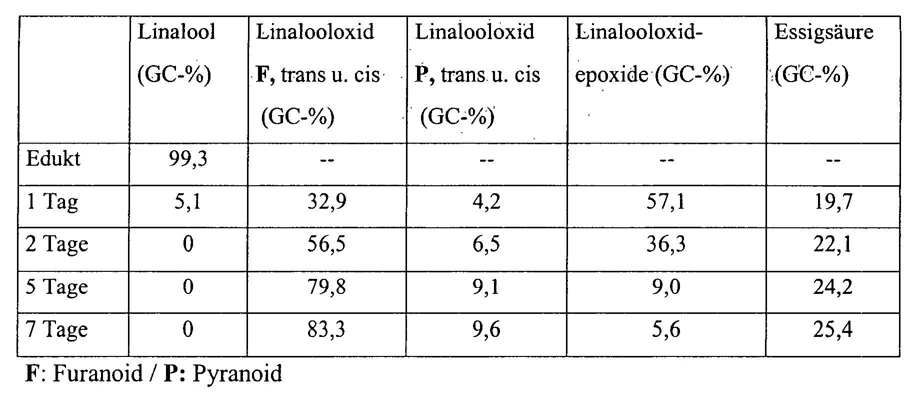

Es wird eine Ausbeute an furanoidem und pyranoidem Linalooloxid von zusammen ca. 93 % erreicht.

Nach destillativer Aufreinigung (10 bödige Kolonne) der Linalooloxid enthaltenden organischen Phase wird ein Produkt mit einer Reinheit von 99 % (GC) furanoidem Linalooloxid erhalten. Dies ist sensorisch in Geschmack und Geruch mit dem naturidentischen Vorbild vergleichbar.

## Patentansprüche

1. Verfahren zur Herstellung von Linalooloxid oder von Linalooloxid enthaltenen Gemischen, **dadurch gekennzeichnet, dass** Linalool oder Linalool enthaltende Gemische in Gegenwart eines Enzyms oxidiert werden.

2. Verfahren nach Anspruch 1, **dadurch gekennzeichnet, dass** D-Linalool, L-Linalool oder etherische Öle, enthaltend D-Linalool oder L-Linalool, oder Gemische dieser Stoffe eingesetzt werden.

3. Verfahren nach einem der Ansprüche 1 oder 2, **dadurch gekennzeichnet, dass** Enzyme aus Hefen oder Bakterien eingesetzt werden.

4. Verfahren nach einem der Ansprüche 1 bis 3, **dadurch gekennzeichnet, dass** Enzyme aus Candida antarctica-Arten, Pseudomonas sp., Humicola sp., Mucor miehei, oder Mischkulturen mit diesen Hefen eingesetzt werden.

5. Verfahren nach einem der Ansprüche 1 bis 4, **dadurch gekennzeichnet, dass** Enzyme aus Candida antarctica eingesetzt werden.

6. Verfahren nach einem der Ansprüche 1 bis 5, **dadurch gekennzeichnet, dass** als Enzyme Lipasen eingesetzt werden.

7. Verfahren nach einem der Ansprüche 1 bis 6, **dadurch gekennzeichnet, dass** die Oxidation in Gegenwart von einem oder mehreren Oxidationsmitteln durchgeführt wird.

8. Verfahren nach einem der Ansprüche 1 bis 7, **dadurch gekennzeichnet, dass** das Oxidationsmittel vor oder während des Verfahrens zugesetzt wird.

9. Verfahren nach einem der Ansprüche 1 bis 8, **dadurch gekennzeichnet, dass** vor oder während des Verfahrens in dem Reaktionsmedium eine Erzeugung des Oxidationsmittels durchgeführt wird.

10. Verfahren nach einem der Ansprüche 1 bis 9 **dadurch gekennzeichnet, dass** das Oxidationsmittel in Gegenwart eines Katalysators vor oder während des Oxidationsverfahrens erzeugt wird.

11. Verfahren nach Anspruch 10, **dadurch gekennzeichnet, dass** als Katalysatoren Enzyme eingesetzt werden.

12. Verfahren nach Anspruch 11, **dadurch gekennzeichnet, dass** als Enzym Glucoseoxidase (GOD) eingesetzt wird.

13. Verfahren nach einem der Ansprüche 7 bis 12, **dadurch gekennzeichnet, dass** das Oxidationsmittel in Mengen von 0,1 bis 80 % dem Reaktionsmedium zugesetzt wird.

14. Verfahren nach einem der Ansprüche 1 bis 13, **dadurch gekennzeichnet, dass** als Oxidationsmittel Wasserstoffperoxid eingesetzt wird.

15. Verfahren nach einem der Ansprüche 1 bis 14, **dadurch gekennzeichnet, dass** dem Reaktionsmedium Lösungsmittel zugesetzt werden.

16. Verfahren nach einem der Ansprüche 1 bis 15, **dadurch gekennzeichnet, dass** als Lösungsmittel Carbonsäure oder Carbonsäureester zugesetzt werden.

17. Verfahren nach einem der Ansprüche 1 bis 16, **dadurch gekennzeichnet, dass** als Lösungsmittel Essigsäureethylester oder Essigsäure eingesetzt werden.

18. Linalooloxid hergestellt durch ein Verfahren nach einem der Ansprüche 1 bis 15.

19. Verwendung des Linalooloxids nach Anspruch 18 als natürlicher Aromastoff für Lebensmittel und für Anwendungen als Riechstoff.
